# EUROPEAN PATENT SPECIFICATION

(11) **EP 1 723 909 B1**
(45) Date of publication and mention of the grant of the patent: **27.10.2010**
(21) Application number: 06113676.8
(22) Date of filing: 09.05.2006
(51) Int. Cl.: A61B 5/22

(54) **Peripheral device of user-specific performance monitor, user-specific performance monitor, and method**
Peripheriegerät zur Leistungsmessung (Pulsuhr), Leistungsmessgerät und Methode
Dispositif périphérique pour la mesure de la performance d'un human, et la méthode associée

(30) Priority: 20.05.2005 FI 20055240
(43) Date of publication of application: 22.11.2006
(73) Proprietor: Polar Electro Oy, 90440 Kempele (FI)
(72) Inventor: Putila, Veli-Pekka, FI-90650, Oulu (FI); Uhari, Ilkka, FI-90570, Oulu (FI); Juola, Petteri, 88610 Vuokatti (FI); Pekonen, Elias, FI-90100, Oulu (FI)
(74) Representative: Antila, Harri Jukka Tapani

(56) References cited:
- EP-A- 0 761 163
- EP-A- 0 785 497
- EP-A- 1 619 475
- WO-A1-2004/032736
- WO-A2-01/78831
- US-A1- 2003 004 403
- US-A1- 2005 075 553
- US-A1- 2005 277 814
- US-B1- 6 738 670

## Description

### FIELD

The invention relates to a peripheral device of a user-specific performance monitor, a user-specific performance monitor, and a method for changing the state of a peripheral device of a user-specific performance monitor.

### BACKGROUND

A user-specific performance monitor is typically an electronic device comprising a user-portable main unit, such as a wrist device, and one or more peripheral devices. The peripheral device may be a device to be fitted on the user's body or in the training environment, producing performance information characterizing the user's performance, the information being transmitted wirelessly to the main unit of the user-specific performance monitor. The purpose of the main unit is usually to process, register and/or display performance information transmitted by the peripheral device or performance-related information generated on the basis of the performance information to the user.

A power source for the peripheral device is typically a battery, the life time of which depends on the active operating time of the peripheral device.

In the prior art solutions, the peripheral device comprises a mechanical switch, by which the user may switch on and off the operating state of the peripheral device. Wireless communication in platforms for biological monitoring has been disclosed in US 2003/0004403 and WO 2004/032736.

In the prior art solutions, the mechanical switch is subjected to mechanical load and impurities, which shorten the age of the mechanical switch and impair its reliability and comfortableness. Hence, it is useful to examine alternative ways of changing the state of the peripheral device.

### BRIEF DESCRIPTION

It is an object of the invention to implement an improved peripheral device of a user-specific performance monitor, a user-specific performance monitor, and a method.

According to an aspect of the present invention, there is provided a peripheral device of a user-specific performance monitor as specified in claim 1.

According to another aspect of the present invention, there is provided a user-specific performance monitor as specified in claim 7.

According to another aspect of the present invention, there is provided a method as specified in claim 8.

The invention is based on the idea that the peripheral device receives a signal wirelessly, the signal level of which is determined and compared with a threshold value. On the basis of the comparison, the state of the peripheral device may be changed between standby and operating states.

The peripheral device of a user-specific performance monitor, the user-specific performance monitor and the method of the invention provide a plurality of advantages. The invention may replace mechanical switches and provide the peripheral device with a sealed capsule structure. In addition, because of the invention the use of the peripheral device is comfortable, since, according to an embodiment, the change between standby and operating states may be implemented by bringing the peripheral device into proximity of a transmitter of the performance monitor, whereupon the signal level of the received signal reaches the threshold value.

### LIST OF FIGURES

The invention will now be described in closer detail in association with the preferred embodiments, with reference to the attached drawings, in which
Figure 1 shows an example of an embodiment of a user-specific performance monitor,
Figure 2 shows an example of an embodiment of a peripheral device of the user-specific performance monitor,
Figure 3 shows an example of a structure of the peripheral device of the user-specific performance monitor,
Figure 4 shows an example of a method according to an embodiment of the shown solution, and
Figure 5 shows a second example of a method according to an embodiment of the shown solution.

### DESCRIPTION OF EMBODIMENTS

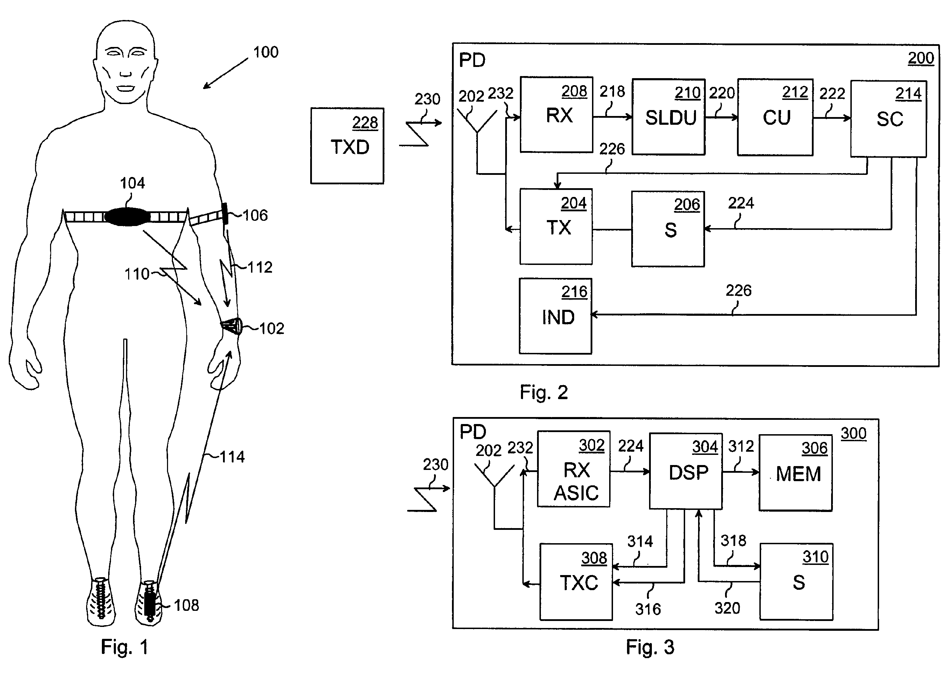

With reference to Figure 1, let us examine an example of an embodiment of a user-specific performance monitor, in which the user-specific performance monitor comprises a main unit 102 and at least one peripheral device 104, 106, 108, which produces performance information characterizing the performance of a user 100 and transmits performance information wirelessly by means of a data transmission signal 110, 112, 114 to the main unit 102.

The data transmission signal 110 to 114 is an electromagnetic signal, for instance. In an embodiment, the frequency of the electromagnetic data transmission signal 110 to 114 is 5 kHz. In another embodiment, the frequency of the electromagnetic data transmission signal 110 to 114 is 2.4 GHz. The above solution is not, however, restricted to these frequencies of the electromagnetic signal, but the frequency may be selected freely according to the embodiment and/or external restrictions.

The performance of the user 100 may be a physical or a mental performance. The quality of the performance as such is not relevant to the shown solution, provided that the performance can be characterized by means of the peripheral device 104, 106, 108.

It is typical of a user-specific performance monitor that the actual user 100 whose performance is being measured monitors the user-specific performance monitor and gives the necessary operation commands of the user-specific performance monitor. In this context, the user-specific performance monitor is shortly referred to as a performance monitor.

The peripheral device 104, 106, 108 is typically an electronic device which characterizes the performance and transmits performance information wirelessly to the main unit 102. Performance information is typically information depending on the performance, whereby its property characterizing the performance may be formed by combining the performance information with some other information characterizing the performance. An example is a peripheral device, which is attached to the training environment, such as a running track, and from which the main unit 102 receives an indicator signal when the main unit 102 enters the proximity of the peripheral device. Thus, the indicator signal implicitly contains position information characterizing the exercise, which may be combined with other exercise-related information, such as heart rate information.

In one configuration the peripheral device 104 is an ECG transmitter (ECG, electrocardiogram), which registers the electrocardiogram of the user 100 and transmits performance information by means of the data transmission signal 110. Thus, the performance information typically contains part of the electrocardiogram of the user 100 and parameters characterizing the electrocardiogram.

In one configuration the peripheral device 106 is a positioning device, such as a GPS positioning device (Global Positioning System), which transmits performance information by means of the data transmission signal 112, the performance information comprising, for instance, the location, speed and/or height position of the user 100. In addition, the performance information may comprise the distance the user 100 has travelled as well as his route and/or direction.

In one configuration the peripheral device 108 is a movement sensor, which transmits the performance information by means of the data transmission signal 114. In this case, the performance information includes movement information on the user 100, such as speed and/or acceleration. The movement sensor may be a shoe sensor, which may be connected or integrated into a shoe of the user 100. In an embodiment, the shoe sensor is a running sensor.

In one configuration the peripheral device is a measuring device which is used in connection with a training means, such as a bicycle and/or exercise bicycle, and which measures parameters of the performance to be carried out by means of the training means, e.g. training force, training power, training speed or other physical variables, and transmits the parameters to the main unit 102.

The peripheral device 104 to 108 is not restricted to the above-mentioned examples, but the peripheral device 104 to 108 may be any electronic device characterizing the performance and transmitting performance information wirelessly to the main unit 102.

The main unit 102 receives the data transmission signal 110 to 114 and processes the performance information. Processing may comprise digital processing of the performance information e.g. by means of calculation algorithms, storage of the performance information, definition of the parameters describing the performance, and displaying the parameter values describing the performance to the user. Parameters describing the performance may include heart rate parameters, energy variables, user speed and/or user activity. The main unit 102 typically comprises a receiver for receiving the data transmission signal 110 to 114, a digital processor for processing the performance information, a memory for storing training-related data and coded instructions to be carried out in the performance monitor, and a user interface for displaying the performance information to the user 100 and inputting commands to the performance monitor. In an embodiment, the main unit 102 comprises a transmitter for transmitting the data transmission signals to the peripheral device 104 to 108.

In one configuration, the main unit 102 is a wrist device to be fastened to the wrist of the user.

In another configuration, the main unit 102 is provided with a quick adapter, by means of which the main unit 102 may be fixed to a desired location, such as a bicycle, structure of an exercise apparatus and/or socket placed on the user's body.

In one configuration, the main unit 102 is integrated in connection with the peripheral device, such as an ECG transmitter belt to be fixed around the chest. In this case, the main unit 102 does not necessarily have a user interface, which may be replaced by means of a separate server by using a wireless data transmission connection between the server and the main unit 102.

With reference to Figure 2, let us examine an example of the structure of a peripheral device (PD) 200. The peripheral device 200 typically comprises a sensor (S) 206, a transmitter unit (TX) 204 connected to the sensor 206, and an antenna unit 202 connected to the transmitter unit 204.

The peripheral device 200 receives a signal 230 wirelessly from a transmitter 228 of the performance monitor. The transmitter 228 may be located in the main unit 102 of the performance monitor and/or in another peripheral device 104 to 108. In an embodiment, the peripheral device 100 is a running sensor 108, and the transmitter 228 is an ECG transmitter 104.

If the transmitter 228 is in a peripheral device, the peripheral device provided with the transmitter 228 need not have the features of the peripheral device 200 of Figure 2, but it is sufficient that the peripheral device provided with the transmitter 228 comprises the transmitter 228.

The sensor 206 typically determines the primary physical variable describing the performance and produces performance information on the basis of the physical variable. The physical variable is, for instance, an ECG signal, a reading on an acceleration sensor or a signal of a satellite positioning system. The sensor 206 may comprise a digital processor for processing the primary measurement information and a memory for storing it.

The sensor 206 may supply a signal containing performance information to the transmitter unit 204, which carries out the processing of the data transmission signal 110 to 114, such as coding, filtering and/or modulation. The transmitter unit 204 converts the data transmission signal 110 to 114 to a data transmission frequency, adjusts the desired power for the data transmission signal 110 to 114 and supplies the data transmission signal 110 to 114 to the antenna unit 202.

The antenna unit 202 converts the data transmission signal 110 to 114 into an electromagnetic field, which is detected in the main unit 102. The antenna unit 202 may comprise a dipole antenna or coil, for example.

The peripheral device 200 of Figure 2 also comprises a receiver unit (RX) 208 connected to the antenna unit 202 and intended for receiving the signal 230, and a signal level determination unit (SLDU) 210 connected to the receiver unit 208 and intended for determining the signal level of the signal 230. In addition, the peripheral device 200 comprises a comparison unit (CU) 212 connected to the signal level determination unit 210 and intended for comparing the signal level of the signal 230 with a threshold value, and a state controller (SC) 214 connected to the comparison unit 212 and intended for changing the state of the peripheral device after the signal level of the signal 230 has reached the threshold value.

The receiver unit 208 comprises a receiver for receiving an electric signal from the antenna unit 202. The antenna unit 202 may be an antenna unit other than the one connected to the transmitter unit 204. The antenna unit may be a dipole antenna and/or coil, for example.

In one configuration, the signal 230 is an electromagnetic signal of 5 kHz, in which case the antenna unit 202 is typically a coil. In another embodiment, the signal 230 is a radio signal of 2.4 GHz, and the antenna unit is typically a dipole antenna. The shown solution is not, however, restricted to these frequencies or antenna types, but the frequency of the signal 230 may be selected from any allowable frequency band.

The receiver unit 208 typically comprises a receiver amplifier, such as a transistor, which amplifies an antenna signal 232 supplied from the antenna unit 202 to the receiver unit 208. The receiver unit 208 may also comprise filters, such as radio frequency filters, frequency converters for converting the radio frequency of the antenna signal 232 to the baseband frequency used by the peripheral device 200, and/or analog/digital converters for sampling the antenna signal 232.

The receiver unit 208 typically produces a receive signal 218 which is proportional to the momentary signal level of the antenna signal 232 and is supplied to the signal level determination unit 210. The signal level determination unit 210 determines the signal level of the receive signal 218, for instance, by rectifying the receive signal 218 in a rectifier unit and integrating the momentary receive signal 218 with respect to time in an integrator. The rectifier unit may be active or passive. An active rectifier unit may be based on a transistor structure, frequency multiplier structure or frequency mixer structure. A passive rectifier unit may be a semiconductor junction or diode, for instance.

The signal level of the signal 230 typically characterizes the power of the signal 230 when the signal 230 is received in the peripheral device 200. The signal level is affected, for instance, by the effective distance between the peripheral device 200 and the transmitter 228, and the transmission power of the signal 230 in the transmitter 228. The effective distance is affected, for instance, by the physical distance between the peripheral device 200 and the transmitter 228, and the electromagnetic environment of the peripheral device 200 and the transmitter 228.

Integration may be carried out calculationally by utilizing digital signal processing, whereby the integration is directed to the digital receive signal 218. Integration may also be carried out analogously by means of analog circuits. In this case, the receive signal 218 is typically an analog signal. In analog integration, the integration may also be directed to the radio-frequency receive signal 218.

The signal level determination unit 210 produces a level signal 220 characterizing the signal level of the signal 230. The level signal 220 may comprise a parameter characterizing the signal level of the signal 230 in numeric form. Thus, the level signal may comprise a bit sequence containing information on the average reception power of the signal 230.

In one configuration, the level signal 220 comprises the signal level of the signal 230 in analog signal form, e.g. as a voltage level, current level and/or frequency.

The level signal 220 is supplied to the comparison unit 212, which compares the signal level information included in the level signal 220 with a threshold value variable characterizing the threshold value of the signal 230. The comparison unit 212 outputs a result signal 222, which contains the result of the comparison between the signal level and the threshold value.

If the level signal 220 contains the signal level information in numeric form, the comparison may be carried out arithmetically, in which case the threshold value variable has been programmed numerically into the comparison unit. An arithmetic comparison may be performed, for instance, by means of a subtraction and/or division in the digital signal processor. The shown solution is not, however, restricted to the arithmetic operations presented above. The result signal 222 contains, for instance, 0 when the signal level is below the threshold value, and 1 when the signal level is above the threshold value.

If the level signal 220 contains the signal level information in analog form, the comparison may be performed by means of a comparator, for instance. In this case, the threshold value variable is voltage level, for example. The comparator may be implemented, for example, by means of a differential amplifier, the first input of which is supplied with a voltage characterizing the signal level and the second input of which is supplied with a voltage corresponding to the threshold value. The comparator outputs a voltage, which may be positive or negative, depending on the magnitude of the difference between the signal level and the threshold value.

The result signal 222 is supplied to the state controller 214, which changes the state of the peripheral device between standby and operating states after the signal level reaches the threshold value.

The threshold value is typically set in such a manner that the signal level does not reach the threshold value unintentionally in the normal use of the performance monitor.

In the normal operation of the performance monitor, the typical minimum distance between the peripheral device 200 and the transmitter 228 and the transmission power of the transmitter 228 are known. The threshold value may be set in such a manner that the reception power of the signal 230 remains below the threshold value, and no change between standby and operating states occurs.

In one configuration, the threshold value is set in such a manner that the signal 230 reaches the threshold value when the peripheral device 200 is brought to a predetermined distance from the transmitter 228. The predetermined distance may be, for instance, 0 to 20 cm but the distance may be selected according to the usual distance between the transmitter 228 and the peripheral device 200. For instance, when the transmitter 228 is an ECG transmitter 104 and the peripheral device is a movement sensor 108, the usual distance is more than a metre. Thus, the threshold value can be reached, for instance, by a distance of 20 cm between the transmitter 228 and the peripheral device 200. However, the invention is not restricted to the fact that the signal level sensed by the peripheral device increases as the distance between the transmitter 228 and the peripheral device 200 decreases, but the signal level may also exceed the threshold value as a result of a procedure performed by the transmitter 228.

The standby state is typically a powersave mode, in which the peripheral device 200 is on standby and can be taken into use quickly. In the standby state, the principal power consumers of the peripheral device 200, such as the transmitter unit 204, the sensor 206 and a potential digital signal processor, are inactive. The typical power consumption of the peripheral device 200 in the standby state is in the range of some microamperes, such as 0.1 to 10 µA.

In the standby state, the receiver unit 208, the signal level determination unit 210 and the comparison unit 212 are in the operating state. Depending on the embodiment, the state controller 214 may be in the standby state or switched off. If the state controller 214 is switched off, the signal determination unit 210, the receiver unit 208 and/or the comparison unit may switch on the state controller 214 after receiving the signal. The signal may or may not exceed the threshold value.

In the above manner, the peripheral device 200 may receive the signal 230 and perform the change from the standby state to the operating state. In the operating state the peripheral device 200 typically produces performance information and transmits performance information wirelessly to the main unit 102. Typically, the power consumption in the operating state is in the range of milliamperes, such as 1 to 20 mA.

The state controller 214 receives the result signal 222 and may transmit a control signal 224 to the transmitter circuit 204 and the sensor 206. The control signal 224 sets the transmitter circuit 204 and/or the sensor 206 to the standby and/or operating state.

In the embodiment of the invention, the control signal 224 includes instructions for changing the state of the peripheral device 200 from the standby state to the operating state. Thus, the state controller 214 may keep the peripheral device 200 in the standby state for a predetermined period of time. After the time period has passed, the state controller 214 may perform a change from the operating state back to the standby state. This may be the case, for instance, when, in spite of the change to the operating state, the peripheral device 200 does not identify the sensor signal, which suggests that there was no intention of taking the peripheral device 200 into use. The predetermined time period may be, for instance, 10 seconds or some minutes without being restricted to the shown examples.

In one configuration, the control signal 224 includes instructions for changing the state of the peripheral device 200 from the operating state to the standby state.

In one configuration, the receiver unit 208 identifies the signal to be used in the peripheral device 200 among a group of signals. In some embodiments, the performance monitor comprises a plurality of signal sources, from which possibly only one is used for changing the state. Identification may be based on identifying signal codes, frequency and/or time slot.

Figure 2 also shows indicator means 216, such as a light-emitting component, for indicating the state of the peripheral device to the user. The state controller 214 transmits to the indicator means (IND) 216 a control signal 226, which includes instructions for changing the signal the indicator means 216 have given to the user 100. The light-emitting component is LED (Light Emitting Diode), for instance. In an embodiment, the indicator means 216 indicate the operating state of the peripheral device 200 to the user.

Figure 3 shows an example of a structure of the peripheral device 300. The peripheral device 300 according to the example comprises a receiver circuit (RX ASIC) 302, which may be implemented by means of ASIC (Application-Specific Integrated Circuit).

In an embodiment, the receiver unit 208, the signal level determination unit 210, the comparison unit 212 and the state controller 214 shown in Figure 2 are implemented by means of the receiver circuit 302.

The receiver circuit 302 generates a control signal 224 and transmits the control signal 224 to a digital signal processor (DSP) 304. The digital signal processor 304 may transmit a state change signal 314 of the transmitter to a transmitter circuit (TXC) 308 and/or a state change signal 318 of the sensor to a sensor 310. After the state has changed from the standby state to the operating state, the sensor 310 starts to generate measuring signals 320 and supply them to the digital signal processor 304, which processes the measuring signal 320 and produces performance information. The performance information is transmitted in a performance information signal 316 to the transmitter circuit 308, where it is processed into a form in which it can be supplied to the antenna unit 202.

Parts of the receiver unit 208, signal level determination unit 210, comparison unit 212 and state controller 214 may be implemented in the digital signal processor 304 by using coded instructions 312 obtained from a memory unit 306.

The coded instructions which are stored in a transmission means may be transferred to the memory unit 306. Transmission means include, for instance, memory circuits, optical memory disks, telecommunication signals, optical signals.

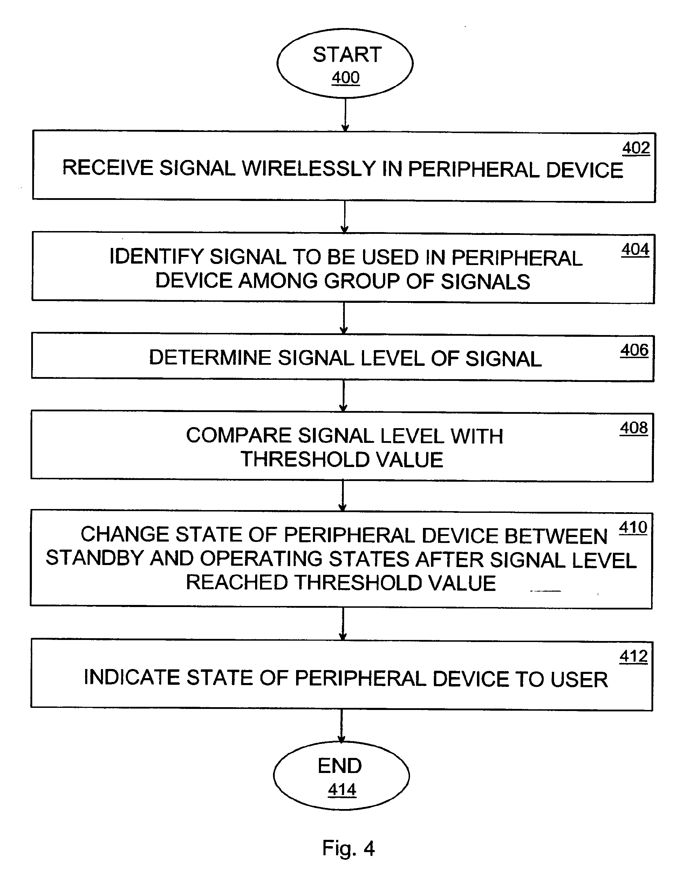

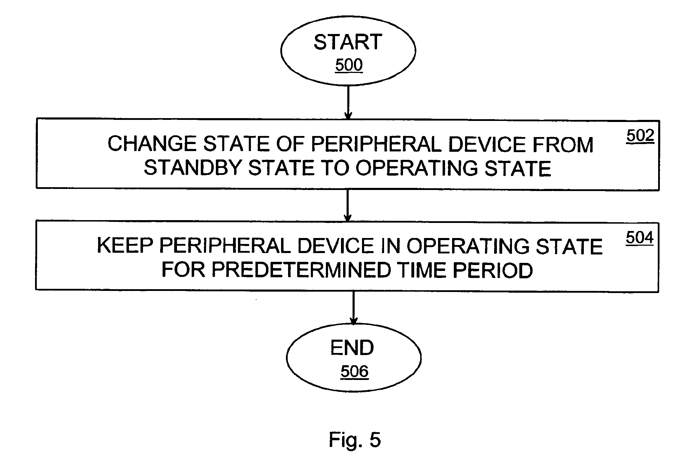

An aspect of the invention is a method for changing the state of a peripheral device of a performance monitor. Figures 4 and 5 show examples of embodiments of the method according to the shown solution.

In 400 of Figure 4, the method starts.

In 402, a signal 230 is received wirelessly in the peripheral device 200.

In 404, the signal to be used in the peripheral device 200 is identified among a group of signals.

In 406, the signal level of the signal 230 is determined.

In 408, the signal level is compared with a threshold value.

In 410, the state of the peripheral device 200 is changed between standby and operating states after the signal level has reached the threshold value.

In 412, the state of the peripheral device 200 is indicated to the user.

In 414, the method ends.

In 500 of Figure 5, the method starts. This stage may be preceded by steps 402 to 408 of Figure 4.

In 502, the state of the peripheral device 200 is changed from the standby state to the operating state after the signal level has reached the threshold value.

In 504, the peripheral device 200 is kept in the operating state for a predetermined period of time.

In 506, the method ends.

Embodiments of the method may be implemented in the digital signal processor 304. Embodiments of the method may be stored as coded instructions in the memory unit 306, and the coded instructions may be transferred to the memory unit 306 by means of transmission means.

Although the invention is described above with reference to the example according to the attached drawings, it is obvious that the invention is not restricted thereto but may be varied in many ways within the scope of the appended claims.

## Claims

1. A peripheral device (200) of a user-specific performance monitor for producing performance information characterizing the user's performance and transmitting it wirelessly to a main unit of the user-specific performance monitor, the peripheral device comprising:
a receiver unit (208) for receiving a signal wirelessly;
a sensor (206) for producing a sensor signal containing performance information characterizing the user's performance;
a signal level determination unit (210) for determining the signal level of the signal, wherein the signal level characterizes the power of the signal;
a comparison unit (212) for comparing the signal level of the signal with a threshold value; and
a state controller (214) for changing the state of the peripheral device between standby and operating states after the signal level has reached the threshold value the state controller (214) being configured to change the state of the peripheral device from the standby state to the operating state after the signal level has reached the threshold value,
**characterized in that** the state controller (214) is further configured to keep the peripheral device in the operating state for a predetermined period of time, and after the time period has passed, to change from the operating state back to the standby state, if the peripheral device does not identify the sensor signal.

2. A peripheral device as claimed in claim 1, wherein the peripheral device also comprises indicator means (216) for indicating the state of the peripheral device to the user.

3. A peripheral device as claimed in claim 1, wherein the peripheral device is a movement sensor for producing movement information on the user.

4. A peripheral device as claimed in claim 3, wherein in that the movement sensor comprises a shoe sensor.

5. A peripheral device as claimed in claim 1, wherein the receiver unit (208) is configured to receive the signal from at least one of the following: another peripheral device of the performance monitor, main unit of the performance monitor.

6. A peripheral device as claimed in claim 1, wherein the receiver unit (208) is arranged to identify the signal to be used in the peripheral device among a group of signals.

7. A user-specific performance monitor comprising:
a main unit (102) for processing the user's performance information; and
at least one peripheral device (200) as claimed in any preceding claim.

8. A method for changing the state of a peripheral device of a user-specific performance monitor, the peripheral device being configured to produce performance information characterizing the user's performance, transmit performance information wirelessly to a main unit of the user-specific performance monitor, and to perform the steps of:
receiving (402) a signal wirelessly in the peripheral device;
producing a sensor signal containing performance information characterizing the user's performance;
determining (406) the signal level of the signal, wherein the signal level characterizes the power of the signal;
comparing (408) the signal level with a threshold value;
changing (410) the state of the peripheral device between standby and operating states after the signal level has reached the threshold value; and
changing (502) the state of the peripheral device from the standby state to the operating state after the signal level has reached the threshold value;
wherein the method further comprises:
keeping (504) the peripheral device in the operating state for a predetermined period of time; and
after the time period has passed, changing from the operating state back to the standby state, if the peripheral device does not identify the sensor signal.

9. A method as claimed in claim 8, with indicating (412) the state of the peripheral device to the user.

10. A method as claimed in claim 8, wherein the peripheral device is a movement sensor for producing movement information on the user.

11. A method as claimed in claim 10, wherein the movement sensor comprises a shoe sensor.

12. A method as claimed in claim 8, with receiving (402) the signal from at least one of the following: another peripheral device of the performance monitor, main unit of the performance monitor.

13. A method as claimed in claim 8, with identifying (404) the signal to be used in the peripheral device among a group of signals.

## Patentansprüche

1. Peripheriegerät (200) eines anwenderspezifischen Leis-Lungsmonitors, welches für die Leistung des Anwenders charakteristische Leistungsinformation erzeugt und sie drahtlos zu einner Haupteinheit des anwenderspezifischen Leistungsmonitors überträgt, wobei das Peripheriegerät aufweist:
eine Empfangseinheit (208) zum Empfangen eines Funksignals;
einen Sensor (206) zur Erzeugung eines Sensorsignales, das Leistungsinformation aufweist, die charakteristisch ist für die Leistung des Anwenders;
eine Signalpegelbestimmungseinheit (210) zum Bestimmen des Signalpegel des Signals, wobei der Signalpegel die Energie des Signals charakterisiert;
eine Vergleichseinheit (212) zum Vergleichen des Signalpegels des Signals mit einem Schwellenwert; und,
eine Zustandssteuerung (214) zum Wechseln des Zustands des Peripheriegeräts zwischen Bereitschafts- und Betriebszustand, nachdem der Signalpegel den Schwellenwert erreicht hat; wobei die Zustandssteuerung (214) konfiguriert ist, den Zustand des Peripheriegeräts aus dem Bereitschaftszustand in den Betriebszustand zu wechseln, nachdem der Signalpegel den Schwellenwert erreicht hat,
**dadurch gekennzeichnet, dass** die Zustandssteuerung (214) ferner konfiguriert ist, um für den Fall, dass das peripheriegerät das Sensorsignal nicht erkennt, das Peripheriegerät für eine vorgegebene Zeitdauer in dem Betriebszustand zu halten und, nachdem die Zeitdauer verstrichen ist, aus dem Betriebszustand in den Bereitschaftszustand zu wechseln.

2. Peripheriegerät nach Anspruch 1, wobei das Peripheriegerät außerdem eine Anzeigeeinrichtung (216) aufweist, um dem Anwender den Zustand des Peripheriegeräts anzuzeigen.

3. Peripheriegerät nach Anspruch 1, wobei das Peripheriegerät ein Bewegungssensor zur Erzeugung von Information über die Bewegung des Anwenders ist.

4. Peripheriegerät nach Anspruch 3, wobei der Bewegungssensor einen Schuhsensor aufweist.

5. Peripheriegerät nach Anspruch 1, wobei die Empfangseinheit (208) konfiguriert ist, das Signal mindestens aus einem der folgenden Geräte zu empfangen: ein anderes Peripheriegerät des Leistungsmonitors, Haupteinheit des Leistungsmonitors.

6. Peripheriegerät nach Anspruch 1, wobei die Empfangseinheit (208) konfiguriert ist, das in dem Peripheriegerät zu verwendende Signal aus einer Gruppe von Signalen zu erkennen.

7. Anwenderspezifischer Leistungsmonitor, aufweisend:
eine Haupteinheit (102) zum Verarbeiten der Anwenderleistungsinformation; und
mindestens ein Peripheriegerät (200) nach einem der vorstehenden Ansprüche.

8. Verfahren zum Wechseln des Zustands eines Peripheriegeräts eines anwenderspezifischen Leistungsmonitors, wobei das Peripheriegerät konfiguriert ist, die Anwenderleistung charakterisierende Leistungsinformation zu erzeugen, die Leistungsinformation drahtlos zu einer Haupteinheit des anwenderspezifischen Leistungsmonitors zu übertragen und die folgenden Schritte auszuführen:
Empfangen (402) eines Funksignals in dem Peripheriegerät;
Erzeugen eines Sensorsignals, das die Anwenderleistung charakterisierende Leistungsinformation aufweist;
Bestimmten (406) des Signalpegels des Signals, wobei der Signalpegel die Energie des Signals charakterisiert;
Vergleichen (408) des Signalpegels mit einem Schwellenwert;
Wechseln (410) des zustands des Peripheriegeräts zwischen Bereitschafts- und Betriebszustand, nachdem der Signalpegel den Schwellenwert erreicht hat; und
Wechseln (502) des Zustands des Peripheriegeräts aus dem Bereitzustand in den Betriebszustand, nachdem der Signalpegel den Schwellenwert erreicht hat;
wobei das Verfahren ferner aufweist:
Halten (504) des Peripheriegeräts in dem Betriebszustand für eine vorgegebene Zeitdauer; und,
nachdem die Zeitdauer verstrichen ist, Wechseln aus dem Betriebszustand zurück in den Bereitschaftszustand, für den Fall, dass das Peripheriegerät das Sensorsignal nicht erkennt.

9. Verfahren nach Anspruch 8, wobei dem Anwender der Zustand des Peripheriegeräts angezeigt wird (412).

10. Verfahren nach Anspruch 8, wobei das Peripheriegerät ein Bewegungssensor zur Erzeugung von Information über die Bewegung des Anwenders ist.

11. Verfahren nach Anspruch 10, wobei der Bewegungssensor einen Schuhsensor aufweist.

12. Verfahren nach Anspruch 8, mit Empfangen (402) des Signals aus mindestens einem der folgenden Geräte: ein anderes Peripheriegerät des Leistungsmonitors, Haupteinheit des Leistungsmonitors.

13. Verfahren nach Anspruch 8, mit Erkennen (404) des in dem Peripheriegerät zu verwendenden Signals aus einer Gruppe von Signalen.

## Revendications

1. Dispositif périphérique (200) d'un moniteur de performance spécifique de l'utilisateur pour produire une information de performance caractérisant la performance de l'utilisateur et la transmettre sans fil à une unité principale d'un moniteur de performance spécifique de l'utilisateur, le dispositif périphérique comprenant :
■ une unité de récepteur (208) pour recevoir un signal sans fil ;
■ un capteur (206) pour produire un signal de capteur contenant une information de performance caractérisant la performance de l'utilisateur ;
■ une unité de détermination de niveau de signal (210) pour déterminer le niveau de signal du signal, où le niveau de signal caractérise la puissance du signal ;
■ une unité de comparaison (212) pour comparer le niveau de signal du signal avec une valeur de seuil ; et
■ une unité de commande d'état (214) pour changer l'état du dispositif périphérique entre des états de pause et de fonctionnement après que le niveau de signal a atteint la valeur de seuil, l'unité de commande d'état (214) étant configurée pour changer l'état du dispositif périphérique de l'état de veille à l'état de fonctionnement après que le niveau de signal a atteint la valeur de seuil,
**caractérisé en ce que** l'unité de commande d'état (214) est en outre configurée pour maintenir le dispositif périphérique dans l'état de fonctionnement pendant un durée prédéterminée, et après que la durée s'est écoulée, pour repasser de l'état de fonctionnement à l'état de veille, si le dispositif périphérique n'identifie pas le signal de capteur.

2. Dispositif périphérique selon la revendication 1, dans lequel le dispositif périphérique comprend également un moyen indicateur (216) pour indiquer l'état du dispositif périphérique à l'utilisateur.

3. Dispositif périphérique selon la revendication 1, dans lequel le dispositif périphérique est un capteur de mouvement pour produire une information de mouvement sur l'utilisateur.

4. Dispositif périphérique selon la revendication 3, dans lequel le capteur de mouvement comprend un capteur de chaussure.

5. Dispositif périphérique selon la revendication 1, dans lequel l'unité de récepteur (208) est configurée pour recevoir le signal d'au moins un des éléments suivants : un autre dispositif périphérique du moniteur de performance, et une unité principale du moniteur de performance.

6. Dispositif périphérique selon la revendication 1, dans lequel l'unité de récepteur (208) est agencée pour identifier le signal à utiliser dans le dispositif périphérique parmi un groupe de signaux.

7. Moniteur de performance spécifique de l'utilisateur comprenant :
■ une unité principale (102) pour traiter l'information de performance de l'utilisateur ; et
■ au moins un dispositif périphérique (200) tel que revendiqué dans l'une quelconque des revendications précédentes.

8. Procédé pour changer l'état d'un dispositif périphérique d'un moniteur de performance spécifique de l'utilisateur, le dispositif périphérique étant configuré pour produire une information de performance caractérisant la performance de l'utilisateur, transmettre l'information de performance sans fil à une unité principale du moniteur de performance spécifique de l'utilisateur, et pour effectuer les étapes consistant à :
■ recevoir (402) un signal sans fil dans le dispositif périphérique ;
■ produire un signal de capteur contenant l'information de performance caractérisant la performance de l'utilisateur ;
■ déterminer (406) le niveau de signal du signal, où le niveau de signal caractérise la puissance du signal ;
■ comparer (408) le niveau de signal avec une valeur de seuil ;
■ changer (410) l'état du dispositif périphérique entre les états de veille et de fonctionnement après que le niveau de signal a atteint la valeur de seuil ; et
■ changer (502) l'état du dispositif périphérique de l'état de veille à l'état de fonctionnement après que le niveau de signal a atteint la valeur de seuil ;
où le procédé comprend en outre les étapes consistant à :
■ maintenir (504) le dispositif périphérique dans l'état de fonctionnement pendant une durée prédéterminée ; et
■ après que la durée s'est écoulée, repasser de l'état de fonctionnement à l'état de veille, si le dispositif périphérique n'identifie pas le signal de capteur.

9. Procédé selon la revendication 8, avec indication (412) de l'état du dispositif périphérique à l'utilisateur.

10. Procédé selon la revendication 8, dans lequel le dispositif périphérique est un capteur de mouvement pour produire une information de mouvement sur l'utilisateur.

11. Procédé selon la revendication 10, dans lequel le capteur de mouvement comprend un capteur de chaussure.

12. Procédé selon la revendication 8, avec réception (402) du signal provenant d'au moins un des éléments suivants : un autre dispositif périphérique du moniteur de performance, et une unité principale du moniteur de performance.

13. Procédé selon la revendication 8, avec identification (404) du signal à utiliser dans le dispositif périphérique parmi un groupe de signaux.
